# EUROPEAN PATENT SPECIFICATION

(11) **EP 0 914 043 B1**
(45) Date of publication and mention of the grant of the patent: **01.10.2003**
(21) Application number: 97915180.0
(22) Date of filing: 21.03.1997
(51) Int. Cl.: A01N 63/00, C12N 5/00, C12N 11/02, C12N 11/10, C12N 11/04, A61K 9/48, A61K 35/23

(54) **IMPLANTABLE AGAROSE-COLLAGEN BEADS CONTAINING CELLS WHICH PRODUCE A DIFFUSIBLE BIOLOGICAL PRODUCT, AND USES THEREOF**
IMPLANTIERBARE AGAROSE-KOLLAGENKÜGELCHEN ENTHALTENDE ZELLEN, DIE EIN DIFFUSIONSFÄHIGES BIOLOGISCHES PRODUKT BILDEN UND IHRE VERWENDUNG
CELLULES CONTENANT DES BILLES D'AGAROSE-COLLAGENE IMPLANTABLES, QUI PRODUISENT UN PRODUIT BIOLOGIQUE DIFFUSIBLE, ET LEURS UTILISATIONS

(30) Priority: 03.04.1996 US 625595; 07.11.1996 US 745063
(43) Date of publication of application: 12.05.1999
(73) Proprietor: THE ROGOSIN INSTITUTE, New York, NY 10021 (US)
(72) Inventor: JAIN, Kanti, New York, NY 10021 (US); RUBIN, Albert, L., Englewood, NJ 07631 (US); ASINA, Shirin, New York, NY 10021 (US); SMITH, Barry, New York, NY 10021 (US); STENZEL, Kurt, New York, NY 10021 (US)
(74) Representative: Helbig, Christian, Dr. Dipl.-Biol.
(86) International application number: US9704548
(87) International publication number: WO97036495

(56) References cited:
- WO-A-95/19430
- WO-A-95/28480
- US-A- 4 647 536
- US-A- 4 663 286
- US-A- 4 673 566
- US-A- 4 798 786
- US-A- 4 971 833
- US-A- 5 053 332

## Description

### Field of the Invention

The present invention relates to the encapsulation of cells in agarose and collagen, followed by coating with agarose, therapeutic methods employing the materials cells, and manufacture thereof.

### Background and Prior Art

The encapsulation of various biological materials in biologically compatible materials is a technique that has been used for some time, albeit with limited success. Exemplary of the art are U.S. Patent Nos. 5,227,298 (Weber, et al); 5,053,332 (Cook, et al); 4,997,443 (walthall, et al) ; 4,971,833 (Larsson, et al) ; 4,902,295 (Walthall, et al) ; 4,798,786 (Tice, et al) ; 4,673,566 (Gooser, et al) ; 4,647,536 (Mosbach, et al) ; 4,409,331 (Lim) ; 4,392,909 (Lim) ; 4,352,883 (Lim) ; and 4,663,286 (Tsang, et al). Also of interest is US 5,643,569 (Jain et al.), filed June 7, 1995 and wo A-95/19430. Jain, et al discusses, in some detail, the encapsulation of secretory cells in various bio-compatible materials. As discussed therein, secretory cells are cells which secrete biological products. Generally, secretory cells possess at least some properties of endocrine cells, and may generally be treated as equivalent to cells which are endocrine in nature. The copending application discusses, e.g., the encapsulation of insulin producing cells, preferably in the form of islets, into agarose-collagen beads which have also been coated with agarose. The resulting products are useful in treating conditions where a subject needs insulin therapy, such as diabetes.

The Jain, et al application discusses in some detail, the prior approaches taken by the art in transplantation therapy. These are summarized herein as well.

Five major approaches to protecting the transplanted tissue from the host's immune response are known. All involve attempts to isolate the transplanted tissue from the host's immune system. The immunoisolation techniques used to date include: extravascular diffusion chambers, intravascular diffusion chambers, intravascular ultrafiltration chambers, microencapsulation, and macroencapsulation. All of these methods have failed, however, due to one or more of the following problems: a host fibrotic response to the implant material, instability of the implant material, limited nutrient diffusion across semi-permeable membranes, secretagogue and product permeability, and diffusion lag-time across semi-permeable membrane barriers.

For example, a microencapsulation procedure for enclosing viable cells, tissues, and other labile membranes within a semipermeable membrane was developed by Lim in 1978. (Lim, Research report to Damon Corporation (1978)). Lim used microcapsules of alginate and poly L-lysine to encapsulate the islets of Langerhans. In 1980, the first successful *in vivo* application of this novel technique in diabetes research was reported (Lim, et al., *Science* 210: 908 (1980)). The implantation of these microencapsulated islets of Langerhans resulted in sustaining a euglycemic state in diabetic animals. Other investigators, however, repeating these experiments, found the alginate to cause a tissue reaction and were unable to reproduce Lim et al's results (Lamberti, et al. *Applied Biochemistry and Biotechnology* 10: 101 (1984); Dupuy, et al., *J. Biomed. Material and Res*. 2-2: 1061 (1988); Weber, et al., *Transplantation* 49: 396 (1990); and Doon-shiong, et al., *Transplantation Proceedings* 22: 754 (1990)). The water solubility of these polymers is now considered to be responsible for the limited stability and biocompatibility of these microcapsules *in vivo* (Dupuy, et al. *supra,* Weber et al. *supra,* Doon-shiong, et al., *supra,* and Smidsrod, *Faraday Discussion of Chemical Society 57:* 263 (1974)).

Recently, Iwata et al., (Iwata, et al. *Jour. Biomedical Material and Res.* 26: 967 (1992)) utilized agarose for microencapsulation of allogenic pancreatic islets and discovered that it could be used as a medium for the preparation of microbeads. In their study, 1500-2000 islets were micro- encapsulated individually in 5% agarose and implanted into streptozotocin-induced diabetic mice. The graft survived for a long period of time, and the recipients maintained normoglycemia indefinitely.

Their method, however, suffers from a number of drawbacks. It is cumbersome and inaccurate. For example, many beads remain partially coated and several hundred beads of empty agarose form. Additional time is thus required to separate encapsulated islets from empty beads. Moreover, most of the implanted microbeads gather in the pelvic cavity, and a large number of islets are required in completely coated individual beads to achieve normoglycemia. Furthermore, the transplanted beads are difficult to retrieve, tend to be fragile, and will easily release islets upon slight damage.

A macroencapsulation procedure has also been tested. Macrocapsules of various different materials, such as poly-2-hydroxyethyl-methacrylate, polyvinylchloride-c-acrylic acid, and cellulose acetate were made for the immunoisolation of islets of Langerhans. (See Altman, et al., *Diabetes 35:* 625 (1986); Altman, et al., *Transplantation: American Society of Artificial Internal Organs* 30: 382 (1984); Ronel, et al., *Jour. Biomedical Material Research* 17: 855 (1983) ; Klomp, et al., *Jour. Biomedical Material Research* 17: 865-871 (1983)). In all these studies, only a transitory normalization of glycemia was achieved.

Archer et al., *Journal of Surgical Research* 28: 77 (1980), used acrylic copolymer hollow fibers to temporarily prevent rejection of islet xenografts. They reported long-term survival of dispersed neonatal murine pancreatic grafts in hollow fibers which were transplanted into diabetic hamsters. Recently Lacy et al., *Science* 254: 1782-1784 (1991) confirmed their results, but found the euglycemic state to be a transient phase. They found that when the islets are injected into the fiber, they aggregate within the hollow tube with resultant necrosis in the central portion of the islet masses. The central necrosis precluded prolongation of the graft. To solve this problem, they used alginate to disperse the islets in the fiber. However, this experiment has not been repeated extensively. Therefore, the membrane's function as an islet transplantation medium in humans is questionable.

Thus, there existed a need for achieving secretory cell transplantation, and, in particular, pancreatic islet allograft and xenograft survival without the use of chronic immunosuppressive agents.

In the Jain, et al work discussed *supra,* the inventors reported that encapsulating secretory cells in a hydrophilic gel material results in a functional, non-immunogenic material, that can be transplanted into animals and can be stored for long lengths of time. The encapsulation of the secretory cells provided a more effective and manageable technique for secretory cell transplantation. The encapsulation technique was described as being useful to encapsulate other biological agents, such as enzymes, microorganisms, trophic agents including recombinantly produced trophic agents, cytotoxic agents, and chemotherapeutic agents. The encapsulated biological agents were discussed as being useful to treat conditions known to respond to the biological agent.

The application does not discuss at any length the incorporation of cells which produce diffusible biological materials, the latter being useful in a therapeutic context. A distinction is made herein between secretory cells and cells which produce diffusible biological materials. The former, as per the examples given in the Jain application, refers generally to products such as hormones, cell signalling agents, etc., which are normally considered to be biological "messengers". In contrast, diffusible biological materials refers to materials such as MHC presented peptides, cell expression regulators such as suppressors, promoters, inducers, and so forth. The distinction will be seen in the field of oncology, e.g., as per the following discussion.

Extensive studies in cancer have included work on heterogeneous cell extracts, and various cellular components. Via the use of monoclonal antibodies, the art has identified relevant cancer associated antigens, e.g., GM2, TF, STn, MUC-1, and various epitopes derived therefrom. Current theory postulates that epitopes derived from these various tumor markers complex non-covalently, with MHC molecules, thereby forming an agrotype by specific cytolytic T cells. This mechanism is not unlike various mechanisms involved in the biological response to viral infections. Note in this regard, Van der Bruggen, et al., *Science* 254: 1643-1647 (1991) ; Boon, et al., 5,405,940, and Boon, et al., 5,342,774.

Additional research which parallels the work on identification of so-called cancer epitopes has focused on the regulation of cancer proliferation, such as via suppression or, more generally, biomodulation. See, e.g., Mitchell, *J. Clin. Pharmacol* 32: 2-9 1992); Maclean, et. al., *Can. J. Oncol.* 4: 249-254 (1994). The aim which unites all of these diverse approaches to cancer is the modification of the host's immune response, so as to bring about some improvement in the patient's condition.

Key to all of these approaches is the activity of one or more diffusible biological products which act in concert with other materials to modulate the immune response. Boon, et al. and van der Bruggen, et al., e.g., disclose small peptide molecules. Mitchell discusses larger molecules which function, e.g., as suppressors.

One problem with all therapeutic approaches which employ these materials is the delivery of these in a safe, effective form. This is not easily accomplished. It has now been found, surprisingly, that the techniques of Jain, et al, which were so useful in the development of therapies for conditions requiring secretory cell products can now be used in other areas.

How this is accomplished is the subject of the invention, the detailed description of which follows.

### DETAILED DESCRIPTION OF PREFERRED EMBODIMENTS

### Example 1

This example, and those which follow, employs RENCA cells. These are spontaneous renal adenocarcinoma cells of BALB/C mice, which are widely available, having been maintained in both *in vitro* and *in vivo* cultures. See Franco et al., Cytokine Induced Tumor Immunogenecity, 181-193 (1994).

Samples of frozen RENCA cells were thawed at 37°C, and then placed in tissue culture flasks containing Dulbecco's Modified Medium (D-MEM), which had been supplemented with 10% bovine serum, penicillin (100 u/ml) and streptomycin (50 ug/ml), to give what will be refereed to as "complete medium" hereafter.

Cells were grown to confluency, and then trypsinized, followed by washing with Hank's Balanced Salt Solution, and then with the complete medium referred to *supra.*

In order to determine if the RENCA cells produced tumors efficiently, two BALB/C mice were injected, intraperitoneally, with 10⁶ of these cells. The mice were observed, over a 3-4 week period. Clinically, they appeared healthy for the first two weeks, and exhibited normal activity. Thereafter, the clinical manifestations of cancer became evident. One mouse died after 23 days, and the second, after 25 days. Following death, the mice were examined, and numerous tumors of various size were noted. Some of the tumors exhibited hemorrhaging as well.

A sample of one tumor, taken from one of the mice, was fixed in 10% formalin for later histological examination.

### Example 2

Following the showing that the RENCA cells did grow *in vivo,* studies were carried out to determine if these cells grew in beads in accordance with the invention.

RENCA cells were grown to confluency, as described *supra,* trypsinized, and washed, also as described above. Samples of between 60,000 and 90,000 cells were then prepared. The cells were then centrifuged, at 750 RPMs, and fluid was removed. The cells were then suspended in solutions of 1% atelocollagen, in phosphate buffered saline solution, at a pH of 6.5.

A 1% solution of low viscosity agarose was prepared in minimal essential medium (MEM), maintained at 60°C, and then 100 µl of this were added to the suspension of RENCA cells and atellocollagen, described *supra.* The materials were then transferred, immediately, as a single large droplet, into sterile, room temperature mineral oil. The mixture formed a single, smooth, semi-solid bead. This procedure was repeated to produce a number of beads.

After one minute the beads were transferred to complete medium, as described *supra,* at 37°C. The beads were then washed three times in minimal essential medium containing the antibiotics listed *supra.* The beads were then incubated overnight at 37°C, in a humidified atmosphere of air and 5% CO₂. Following the incubation, the beads, now solid, were transferred to a sterile spoon which contained 1 ml of 5% agarose in minimal essential medium. Beads were rolled in the solution 2-3 times to uniformly coat them with agarose. The beads were transferred to mineral oil before the agarose solidified, to yield a smooth outer surface. After 60 seconds, the beads were washed, five times, with complete medium at 37°C to remove the oil. Overnight incubation (37°C, humidified atmosphere of air, 5% CO₂) followed.

These RENCA containing beads were used in the experiments which follow.

### Example 3

Prior to carrying out *in vivo* investigations, it was necessary to determine if the RENCA cells would grow in the beads prepared in the manner described *supra.*

To do this, beads prepared as discussed in example 2 were incubated in the medium described in example 2, for a period of three weeks under the listed conditions. Three of the beads were then cut into small pieces, and cultured in standard culture flasks, affording direct contact with both the flask and culture medium.

Observation of these cultures indicated that the cells grew and formed standard RENCA colonies. This indicated that the cells had remained viable in the beads.

### Example 4

*In vivo* experiments were then carried out. In these experiments, the beads were incubated for seven days, at 37 °C. Subject mice then received bead transplants. To do this, each of four mice received a midline incision, carried through intraperitoneally. Three beads, each of which contained 60,000 RENCA cells were transplanted. Incisions were then closed (two layer closure), using an absorbable suture. The four mice (BALB/C) were normal, male mice, weighing between 24-26 grams, and appeared to be healthy. Two sets of controls were set up. In the first set, two mice received three beads containing no RENCA cells, and in the second, two mice were untreated with anything.

Three weeks after the implantation, all of the mice received intraperitoneal injections of 10⁶ RENCA cells. Eighteen days later, one control mouse died. All remaining mice were then sacrificed, and observed.

Control mice showed numerous tumors, while the mice which received the implants of bead-encapsulated cells showed only random nodules throughout the cavity.

These encouraging results suggested the design of the experiments set forth in the following example.

### Example 5

In these experiments, established cancers were simulated by injecting RENCA cells under one kidney capsule of each of six BALB/C mice. Fifteen days later, mice were divided into two groups. The three mice in the first group each received three beads, as described in example 4, *supra.* The second group (the control group) received beads which did not contain RENCA cells.

After 4-5 days, mice which had received RENCA cell containing implants looked lethargic, and their fur had become spiky, while the control group remained energetic, with no change in condition of fur.

Ten days after implantation (25 days after injection of RENCA cells), however, the control mice became sluggish, and exhibited distended abdomens. One of the three control mice died at fourteen days following bead transplantation. Sacrifice of the mice followed.

The body cavities of the control mice showed profuse hemorrhaging, with numerous tumors all over the alimentary canal, liver, stomach and lungs. The entire abdominal cavity had become indistinguishable due to rampant tumor growth. The mice which had received beads with encapsulated RENCA cells, however showed no hemorrhaging, and only a few nodules on the alimentary cancers. Comparison of test and control groups showed that, in the test group, nodules had not progressed.

### Example 6

Freely inoculated RENCA cell growth is inhibited when incubated along with encapsulated RENCA cells. A further set of experiments were carried out to determine if this effect was observable with other cells.

An adenocarcinoma cell line, i.e., MMT (mouse mammary tumor), was obtained from the American Type Culture Collection. Encapsulated MMT cells were prepared, as described, *supra* with MMT cells, to produce beads containing 120,000 or 240,000 cells per bead. Following preparation of the beads, they were used to determine if they would inhibit proliferation of RENCA cells *in vitro.* Specifically, two, six well petri plates were prepared, via inoculation with 1x10⁴ RENCA cells per well, in 4 ml of medium. In each plate, three wells served as control, and three as test. One of the three control wells in each plate received one bead. Each of the other wells received either two or three empty beads. The second well was treated similarly, with wells receiving one, two or three beads containing 120,000 or 240,000 MMT cells. Wells were incubated at 37°C for one week, after which RENCA cells were trypsinized, washed, and counted, using a hemocytometer. Results follow:

| DISH # 1 (EMPTY MACROBEADS) # of cells retrieved after one week | | | DISH # 2 (MACROBEADS WITH MMT CELLS) # of cells retrieved after one week | |
|---|---|---|---|---|
| Well # | Control | Empty | 120,000 MMT cells | 240,000 MMT cells |
| 1 | 2.4 x 10⁵ | 2.8 x 10⁵ | 1.4 x 10⁵ | 1 x 10⁵ |
| 2 | 2.0 x 10⁵ | 3.5 x 10⁵ | 1.2 x 10⁵ | 7 x 10⁴ |
| 3 | 4.4 x 10⁵ | 2.5 x 10⁵ | 1.25 x 10⁵ | 9 x 10⁴ |

### Example 7

Following the results in example 6, the same experiments was carried out, using 1x10⁴ MMT cells rather than RENCA cells. The experiment was carried out precisely as example 6. Results are set forth below.

| Well # | Control | Empty Macrobeads | (1) MMT Macrobeads | (2) MMT Macrobeads |
|---|---|---|---|---|
| 1 | 3.1 x 10⁶ | 2.8 x 10⁶ | 1.6 x 10⁶ | 1.3 x 10⁶ |
| 2 | 3.3 x 10⁶ | 2.6 x 10⁶ | 1.0 x 10⁶ | 1.1 x 10⁶ |
| 3 | 3.0 x 10⁶ | 2.8 x 10⁶ | 6.0 x 10⁵ | 5.0 x 10⁵ |
| | | | | |

These results encouraged the use of an *in vivo* experiment. This is presented in example 8.

### Example 8

RENCA cells, as used in the preceding examples, are renal cancer cells. To demonstrate more completely the general efficacy of the invention, work was carried out using a different type of cancer cells. Specifically, adenocarcinoma cells were used.

A mouse mammary tumor cell line (MMT) was obtained from the American Type Culture Collection. Using the protocols set forth, *supra,* implants were prepared which contained 120,000 cells per bead, and 240,000 cells per bead.

The experimental model used was the mouse model, *supra.* Twenty two mice were divided into groups of 4, 9 and 9. The first group, i.e., the controls, were further divided into three groups of two, one and one. The first subgroup received implants of one bead containing no cells. One mouse received two empty beads, and one received three empty beads.

Within experimental group A (9 animals), the beads contained 120,000 cells, while in group B, the beads contained 240,000 cells. Within groups "A" and "B", there were three subdivisions, each of which contained three mice. The subgroups received one, two, or three beads containing MMT cells.

Twenty one days following implantation, all animals received injections of 40,000 RENCA cells. Immediately after injection, the mice were lethargic, with spiky hair. This persisted for about five days, after which normal behavior was observed.

After twenty days, control mice exhibited distended abdomens, and extremely spiky hair. One control mouse died 25 days following injection, while the remaining control mice appeared terminal. All mice were sacrificed, and tumor development was observed. These observations are recorded infra:

| NUMBER OF MACROBEADS IN MICE | CONTROL | EXPERIMENTAL GROUP A | EXPERIMENTAL GROUP B |
|---|---|---|---|
| 1 | ++++ | -- | -- |
| 1 | ++++ | -- | -- |
| 1 | | + | ++ |
| | | | |
| 2 | ++++ | -- | -- |
| 2 | | -- | -- |
| 2 | | ++ | ++ |
| | | | |
| 3 | ++++ | -- | -- |
| 3 | | -- | -- |
| 3 | | -- | +++ |

These results show that, of eighteen mice tested, thirteen showed no disease. Of the mice in Group (A), one mouse exhibited a few modules, and another mouse showed a few tumors. One mouse which received two beads showed a few tumors.

Within group B, one mouse which had received one bead, and one mouse which received two beads showed a few tumors, entangled with intestines. One of the mice which received three beads had developed a large solid tumor and was apparently very sick. Nonetheless, the overall results showed that the encapsulated mouse mammary tumor cells inhibited tumor formation.

### Example 9

As suggested, *supra,* the practice of the invention results in the production of some material or factor which inhibits and/or prevents tumor cell proliferation. This was explored further in the experiment which follows.

Additional beads were made, as described *supra* in example 2, except that atellocollagen was not included. Hence, these beads are agarose/agarose beads. RENCA cells, as described, *supra,* were incorporated into these beads, again as described *supra.*

Two sets of three six well plates were then used as control, and experimental groups. In the control *group,* wells were filled with 4 ml of RPMI complete medium (10% fetal calf serum and 11 ml/l of penicillin). Each control group well was then inoculated with 10,000 RENCA cells.

In the experimental group, the RPMI complete medium was conditioned, by adding material secured by incubating 10 immuno-isolated, RENCA containing beads (120,000 cell per bead), in a 35x100 mm petri plate containing 50 ml of the RPMI complete medium. Following five days of incubation, medium was collected from these plates, and 4 ml of it was placed in each test well. These wells were then inoculated with 10,000 RENCA cells.

All plates (both control and experimental) were incubated at 37°C for five days. Following the incubation period cells were trypsinization washed, and counted using a hemocytometer. The cells in the plates of each well were pooled together following trypsinization, and counted, the results follow.

| WELL # | (CELLS) CONTROL | (CELLS) CONDITIONED |
|---|---|---|
| 1 | 7 x 10⁵ | 3 X 10⁵ |
| 2 | 8 x 10⁵ | 2.5 x 10⁵ |
| 3 | 7 x 10⁵ | 3.4 X 10⁵ |

These results show that the cells, when restricted in, e.g., the beads of the invention, produced some factor which resulted in suppression of tumor cell proliferation. This restriction inhibitor factor is produced by the cells in view of their, entrapment in the bead, and differs from other materials such as contact inhibitor factor, which are produced when cells contact each other.

### Example 10

The experiment set forth *supra* showed that RENCA cell growth, in conditioned medium, was about half the growth of the cells in control medium. The experiments set forth herein examined whether the growth inhibiting factor would remain active after the conditioned medium was frozen.

RENCA conditioned medium was prepared by incubating 10 immunoisolated RENCA containing beads for five days. Incubation was in 35x100 mm petri plates, with 50 ml RMPI complete medium, at 37°C. Following the incubation, the medium was collected and stored at -20 C. Conditioned medium was prepared by incubating immunoisolated MMT (mouse mammary tumor) cell containing beads. The beads contained 240,000 cell per bead; otherwise all conditions were the same.

Frozen media were thawed at 37°C, and then used in the following tests. Three six well plates were used for each treatment, i.e., (i) RMPI control medium, (2) RENCA frozen conditioned medium, and (3) MMT frozen conditioned medium. A total of 4 ml of medium was dispensed into each well. All wells were then inoculated with 10,000 RENCA cells, and incubated at 37°C, for five days. Following incubation, two plates of samples were taken from each well, trypsinized, pooled, and counted in a hemocytometer. At eight days, the remaining three plates of each well were tested in the same way.

Results follow:

| DISH 5 DAYS OLD | CONTROL MEDIUM | FROZEN CONDITIONED MEDIUM OF RENCA | FROZEN CONDITIONED MEDIUM OF MMT |
|---|---|---|---|
| 1 | 6 X 10⁵ | X 10⁵ | 8 X 10⁴ |
| 2 | 6.8 X 10⁵ | 4.2 X 10⁵ | 8.5 X 10⁴ |
| 8 DAYS OLD | | | |
| 3 | 2.8 X 10⁶ | 2 X 10⁶ | 8 X 10⁴ |

When these results are compared to those in example 6, *supra,* it will be seen that, while the frozen/thawed RENCA conditioned medium did not arrest growth to the same extent that unfrozen medium did (compare examples 6 and 7), it did, nonetheless, arrest growth). Frozen conditioned medium using MMT cells arrested growth even more than the unfrozen MMT conditioned medium. These results show that, of eighteeen mice tested, thirteen showed no disease. Of the mice in Group (A), one mouse exhibited a few modules, and another mouse showed a few tumors,

The foregoing describes the manufacture of implantable beads which contain one or more types of cells which produce a diffusible biological product, as this phrase is defined herein. The diffusible biological product is one which has an effect on the subject in which the bead is implanted. Preferably, this effect is immunomodulation, such as stimulating an immune response, or suppressing a response. In the case of cancer, for example, the diffusible biological product may be a peptide which complexes with MHC molecules on cancer cells in a subject, thereby provoking a CTL response thereto in turn leading to lowering of the tumor load in the subject. The diffusible product may also be a suppressor of tumor growth. In connection with this form of therapy, it is possible, although not necessarily preferable, to place the implanted beads in or near an identified tumor.

"Diffusible biological product" as used herein refers to materials such as proteins, glycoproteins, lipoproteins, carbohydrates, lipids, glycolipids, and peptides. More specifically, materials such as antibodies, cytokines, hormones, enzymes, and so forth, are exemplary, but by no means the only type of materials included. Excluded are the well known "end products" of cellular processes, such as CO₂. and H₂O.

As the experiments show, the implantable beads may also be used prophylactically. It is well known that at least a segment of the population of cancer patients are prone to re-occurrence of the condition. The experiments described herein show that the implants can prevent the occurrence or reoccurrence of cancer, via the biological effect the diffusible product has on a subject's system.

The discussion of the invention has focused on *in vivo* approaches. It must also be understood that there are *in vitro* approaches to the invention, some of which are discussed upon herein. For example, it is well known that many cells which produce desirable products, when cultured *in vitro,* require the presence of feeder cells. There are always issues with such feeder cells. They may grow faster than the desired cells, leading to de facto "strangulation" of the materials of interest. Further, there can be a problem with various toxic products being produced by the feeder layers. The implantable beads of the invention act almost as cellular incubators, protecting the incorporated cells, while permitting the diffusible products to move into a culture medium, e.g. , where they can be collected.

As indicated, *supra,* preparation of the implantable beads first requires suspension of the cells in solution, preferably aqueous of collagen. Preferably, the collagen is atelocollagen, in a solution of from about 0.5 to about 2%. Depending upon the type of cell used, the number of cells in the solution at a given time, and hence the number of cells in a bead, will vary. Preferably, there are from about 10,000 to about 200,000 cells used per bead, more preferably from about 30,000 to about 100,000. Most preferably, about 40,000 to about 60,000 cells are used.

Following suspension of the cells in the collagen solution, an agarose solution is added. Preferably, this agarose solution will range from about 0.5% to about 5%, preferably about 1%. By dropping the mixture onto or into inert materials, such as TEFLON® or mineral oil, a bead forms. This bead is semi-solid. The semi-solid bead is then transferred to a sterile medium, preferably one containing antibiotics, washed, and incubated to polymerize collagen. The polymerization of collagen is a well studied phenomenon, and the conditions under which this occurs need not be elaborated upon herein.

Following the solidification of the bead, it is then coated with agarose, preferably by rolling it in an agarose solution. One preferred way of accomplishing this is a simple TEFLON® coated spoon which contains a solution of agarose, preferably 5% to 10%.

The foregoing discussion of diffusible biological products should not be construed as being limited to wild type materials. For example, one can just as easily incorporate transformed or transfected host cells, such as eukaryotic cells (e.g., 293 cells, CHO cells, COS cells), or even prokaryotic cells (e.g., E. coli), which have been treated to produce heterogeneous protein, or modified via, e.g., homologous recombination, to produce increased amounts of desirable biological products, Other materials, such as hybridomas, may also be used, with the diffusible biological product being a monoclonal antibody.

## Claims

1. A composition of matter comprising an agarose coated, solid agarose collagen bead wherein said bead contains cells which when restricted by being entrapped in said bead produce a factor that suppresses tumor cell proliferation, wherein said factor diffuses through said coated, solid agarose collagen bead.

2. The composition of matter of claim 1 wherein said cells are cancer cells, preferably renal cancer cells.

3. The composition of matter of claim 1, wherein said bead contains from about 10,000 to about 200,000 cells and, preferably, from about 30,000 to about 100,00 cells.

4. The composition of matter as claimed in any of claims 1 to 3 for use in the treatment of a pathological condition.

5. The composition of matter as claimed in claim 4 wherein said pathological condition is **characterized by** abnormal cell growth.

6. The composition of matter as claimed in claim 5, wherein said pathological condition is cancer.

7. Method for administering a factor to cells cultured *in vitro* comprising incubating said cells with the composition of matter of any of claims 1 to 3 wherein said factor diffuses through said composition of matter.

8. A process for manufacturing an agarose coated, agarose collagen bead which contains cells which when restricted by being entrapped in said bead produce a factor that suppresses tumor cell proliferation which diffuses through said agarose coated, solid agarose collagen bead, comprising:
suspending cells which when restricted produce the factor in a collagen containing material;
adding agarose to said collagen containing material;
forming a semi-solid bead of said collagen, agarose and cells ;
polymerizing collagen in said semi-solid bead to form a solid, agarose-collagen bead containing said cells; and
coating said solid agarose-collagen bead which contains said cells with agarose.

9. The process of claim 8, wherein said cells are cancer cells.

10. The process of claim 8, comprising suspending form about 10,000 to about 200,000 cells and, preferably, from about 30,000 to about 100,000 cells in said collagen containing solution.

11. A composition of matter comprising a solid, agarose coated, agarose containing bead, wherein said bead contains cells which when restricted by being entrapped in said bead produce a factor that suppresses tumor cell proliferation, wherein said factor diffuses through said solid, agarose coated, agarose containing bead.

12. The composition of matter of claim 11, wherein said restricted cells are cancer cells and, preferably, renal cancer cells.

13. The composition of matter of claim 11, wherein said bead contains from about 10,000 to about 200,000 cells and, preferably, from about 30,000 to about 100,000 cells.

14. The composition of matter as claimed in any of claims 11 to 13 for use in the treatment of a pathological condition.

15. The composition of matter as claimed in claim 14, wherein said pathological condition is **characterized by** abnormal cell growth.

16. The composition of matter as claimed in claim 15, wherein said pathological condition is cancer.

17. Method for administering a factor to cells cultured *in vitro* comprising incubating said cells with the composition of matter of any of claims 11 to 13 wherein after incubation said factor diffuses through said composition of matter.

18. A process for making a solid bead which comprises agarose, and is coated with agarose, wherein said solid bead contains cells, which, when restricted by being entrapped in said bead produce a factor that suppresses tumor cell proliferation which diffuses through said bead comprising:
adding agarose to a solution which contains a sample of cells which are capable of producing a factor that suppresses tumor cell proliferation which diffuses through said bead when said cells are restricted;
forming a semi-solid bead comprising said agarcse and said cells;
polymerizing the agarose in said semi-solid bead to form a solid, agarose bead containing said cells and thereby restricting said cells; and
coating said solid, agarose containing bead containing the restricted cells with agarose.

19. The process of claim 18, wherein said cells are cancer cells.

20. The process of claim 18, comprising suspending from about 10,000 to about 200,000 cells and, preferably, from about 30,000 to about 100,000 cells in said solution.

21. A composition of matter comprising a solid, agarose coated, agarose containing bead, wherein said bead contains cancer cells isolated from an animal which, when restricted by being entrapped in said bead, produce more of a material that suppresses cancer cell proliferation, wherein said material diffuses through said solid, agarose coated, agarose containing bead.

22. The composition of matter of claim 21, wherein said cancer cells are renal cancer cells.

23. The composition of matter of claim 21, wherein said bead contains from about 10,000 to about 200,000 cells.

24. The composition of matter of claim 23, wherein said bead contains from about 30,000 to about 100,000 cells.

25. The composition of matter as claimed in any of claims 21 to 24 for the treatment of a pathological condition which is **characterized by** abnormal cell growth.

26. The composition of matter as claimed in any of claims 21 to 24 for the treatment of a pathological condition which is cancer.

27. A composition of matter as claimed in any of one of claims 21 to 24, for use in suppressing the proliferation of cancer cells in a subject.

28. Use of a composition of matter as claimed in any one of claims 21 to 24 for the manufacture of a medicament for implantation into a subject to suppress the proliferation of cancer cells in said subject.

29. A process for making a solid bead which comprises agarose, and is coated with agarose, wherein said solid bead contains cancer cells which, when restricted by being entrapped in said bead, produce material that suppresses cancer cell proliferation and diffuses through said bead, comprising
(a) adding agarose to a solution which contains a sample of cancer cells isolated from an animal which are capable of producing material that suppresses cancer cell proliferation which diffuses through said bead when said cancer cells are restricted by being entrapped by the bead,
(b) forming a semi-solid bead comprising said agarose and said cancer cells,
(c) polymerizing the agarose in said semi-solid bead to form a solid, agarose bead containing and thereby restricting said cancer cells, and
(d) coating said solid, agarose containing bead containing the restricted cancer cells with agarose, wherein said restricted cancer cells produce more of said material than when said cancer cells are not entrapped in said bead.

30. The process of claim 29, wherein said solution contains from about 10,000 to about 200,000 cells.

31. The process of claim 29, wherein said solution contains from about 30,000 cells to about 100,000 cells.

## Patentansprüche

1. Stoffzusammensetzung, umfassend ein Agarose-beschichtetes, festes Agarose-Kollagen-Kügelchen, wobei das Kügelchen Zellen enthält, welche, wenn durch Einschluss in dem Kügelchen restringiert, einen Tumorzellproliferation supprimierenden Faktor produzieren, wobei der Faktor durch das beschichtete, feste Agarose-Kollagen-Kügelchen diffundiert.

2. Stoffzusammensetzung nach Anspruch 1, wobei die Zellen Krebszellen, bevorzugt Nierenkrebszellen sind.

3. Stoffzusammensetzung nach Anspruch 1, wobei das Kügelchen von etwa 10.000 bis etwa 200.000 Zellen und bevorzugt von etwa 30.000 bis etwa 100.000 Zellen enthält.

4. Stoffzusammensetzung nach einem der Ansprüche 1 bis 3 zur Verwendung bei der Behandlung eines pathologischen Zustands.

5. Stoffzusammensetzung nach Anspruch 4, wobei der pathologische Zustand durch abnormales Zellwachstum gekennzeichnet ist.

6. Stoffzusammensetzung nach Anspruch 5, wobei der pathologische Zustand Krebs ist.

7. Verfahren zur Verabreichung eines Faktors Zellen, die in vitro kultiviert werden, umfassend das Inkubieren der Zellen mit der Stoffzusammensetzung nach einem der Ansprüche 1 bis 3, wobei der Faktor durch die Stoffzusammensetzung diffundiert.

8. Verfahren zur Herstellung eines Agarose-beschichteten, Agarose-Kollagen-Kügelchens, das Zellen enthält, welche, wenn durch Einschluss in dem Kügelchen restringiert, einen Tumorzellproliferation supprimierenden Faktor produzieren, wobei der Faktor durch das Agarose-beschichtete, feste Agarose-Kollagen-Kügelchen diffundiert, umfassend:
Suspendieren von Zellen, die, wenn restringiert, den Faktor produzieren, in einem Kollagen-enthaltenden Material,
Zugeben von Agarose zu dem Kollagen-enthaltenden Material,
Bilden eines semi-festen Kügelchens aus dem Kollagen, der Agarose und den Zellen,
Polymerisieren von Kollagen in dem semi-festen Kügelchen, wobei ein festes, die Zellen enthaltendes Agarose-Kollagen-Kügelchen gebildet wird, und
Beschichten des festen, die Zellen enthaltenden Agarose-Kollagen-Kügelchens mit Agarose.

9. Verfahren nach Anspruch 8, wobei die Zellen Krebszellen sind.

10. Verfahren nach Anspruch 8, umfassend das Suspendieren von etwa 10.000 bis etwa 200.000 Zellen und bevorzugt von etwa 30.000 bis etwa 100.000 Zellen in der Kollagen-enthaltenden Lösung.

11. Stoffzusammensetzung, umfassend ein Agarose-beschichtetes, festes Agarose-enthaltendes Kügelchen, wobei das Kügelchen Zellen enthält, welche, wenn durch Einschluss in dem Kügelchen restringiert, einen Tumorzellproliferation supprimierenden Faktor produzieren, wobei der Faktor durch das Agarose-beschichtete, feste Agarose-enthaltende Kügelchen diffundiert.

12. Stoffzusammensetzung nach Anspruch 11, wobei die restringierten Zellen Krebszellen, und bevorzugt Nierenkrebszellen sind.

13. Stoffzusammensetzung nach Anspruch 11, wobei das Kügelchen von etwa 10.000 bis etwa 200.000 Zellen und bevorzugt von etwa 30.000 bis 100.000 Zellen enthält.

14. Stoffzusammensetzung nach einem der Ansprüche 11 bis 13 zur Verwendung bei der Behandlung eines pathologischen Zustands.

15. Stoffzusammensetzung nach Anspruch 14, wobei der pathologische Zustand durch abnormales Zellwachstum gekennzeichnet ist.

16. Stoffzusammensetzung nach Anspruch 15, wobei der pathologische Zustand Krebs ist.

17. Verfahren zur Verabreichung eines Faktors Zellen, die in vitro kultiviert werden, umfassend das Inkubieren der Zellen mit der Stoffzusammensetzung nach einem der Ansprüche 11 bis 13, wobei nach Inkubation der Faktor durch die Stoffzusammensetzung diffundiert.

18. Verfahren zur Herstellung eines festen Kügelchens, das Agarose enthält und mit Agarose beschichtet ist, wobei das feste Kügelchen Zellen enthält, welche, wenn durch Einschluss in dem Kügelchen restringiert, einen Tumorzellproliferation supprimierenden Faktor produzieren, der durch das Kügelchen diffundiert, umfassend:
Zugeben von Agarose zu einer Lösung, die eine Probe von Zellen enthält, welche einen Tumorzellproliferation supprimierenden Faktor produzieren können, der durch die Kügelchen diffundiert, wenn die Zellen restringiert sind,
Bilden eines semi-festen Kügelchens, umfassend die Agarose und die Zellen,
Polymerisieren der Agarose in dem semi-festen Kügelchen, wobei ein festes, die Zellen enthaltendes Agarosekügelchen gebildet wird und die Zellen dadurch restringiert werden, und
Beschichten des festen, Agarose-enthaltenden Kügelchens, das die restringierten Zellen enthält, mit Agarose.

19. Verfahren nach Anspruch 18, wobei die Zellen Krebszellen sind.

20. Verfahren nach Anspruch 18, umfassend das Suspendieren von etwa 10.000 bis etwa 200.000 Zellen und bevorzugt von etwa 30.000 bis etwa 100.000 Zellen in der Lösung.

21. Stoffzusammensetzung, umfassend ein Agarose-beschichtetes, festes Agarose-enthaltendes Kügelchen, wobei das Kügelchen aus einem Tier isolierte Krebszellen enthält, welche, wenn durch Einschluss in dem Kügelchen restringiert, mehr eines Tumorzellproliferation supprimierenden Materials produzieren, wobei das Material durch das Agarose-beschichtete, feste Agarose-enthaltende Kügelchen diffundiert.

22. Stoffzusammensetzung nach Anspruch 21, wobei die Krebszellen Nierenkrebszellen sind.

23. Stoffzusammensetzung nach Anspruch 21, wobei das Kügelchen von etwa 10.000 bis etwa 200.000 Zellen enthält.

24. Stoffzusammensetzung nach Anspruch 21, wobei das Kügelchen von etwa 30.000 bis etwa 100.000 Zellen enthält.

25. Stoffzusammensetzung nach einem der Ansprüche 21 bis 24 für die Behandlung eines pathologischen Zustandes, der durch abnormales Zellwachstum gekennzeichnet ist.

26. Stoffzusammensetzung nach einem der Ansprüche 21. bis 24 für die Behandlung eines pathologischen Zustandes der Krebs ist.

27. Stoffzusammensetzung nach einem der Ansprüche 21 bis 24, zur Verwendung bei der Suppression der Proliferation von Krebszellen in einem Individuum.

28. Verwendung einer Stoffzusammensetzung nach einem der Ansprüche 21 bis 24 zur Herstellung eines Arzneimittels zur Implantation in ein Individuum, um die Proliferation von Krebszellen in dem Individuum zu supprimieren.

29. Verfahren zur Herstellung eines festen Kügelchens, das Agarose enthält und mit Agarose beschichtet ist, wobei das feste Kügelchen Krebszellen enthält, welche, wenn durch Einschluss in dem Kügelchen restringiert, Tumorzellproliferation supprimierendes Material produzieren, welches durch das feste Kügelchen diffundiert, umfassend:
(a) Zugeben von Agarose zu einer Lösung, die eine Probe von aus einem Tier isolierten Krebszellen enthält, welche ein Tumorzellproliferation supprimierendes Material produzieren können, das durch die Kügelchen diffundiert, wenn die Krebszellen durch Einschluss in dem Kügelchen restringiert sind,
(b) Bilden eines semi-festen Kügelchens, umfassend die Agarose und die Krebszellen,
(c) Polymerisieren der Agarose in dem semi-festen Kügelchen, wobei ein festes, die Zellen enthaltendes Agarosekügelchen gebildet wird und die Krebszellen dadurch restringiert werden, und
(d) Beschichten des festen, Agarose-enthaltenden Kügelchens, das die restringierten Krebszellen enthält, mit Agarose, wobei die restringierten Krebszellen mehr von dem Material produzieren, als wenn die Krebszellen nicht in dem Kügelchen eingeschlossen sind.

30. Verfahren nach Anspruch 29, wobei die Lösung von etwa 10.000 bis etwa 200.000 Zellen enthält.

31. Verfahren nach Anspruch 29, wobei die Lösung von etwa 30.000 Zellen bis etwa 100.000 Zellen enthält.

## Revendications

1. Composition de matière comprenant une bille solide d'agarose -collagène enduite d'agarose dans laquelle ladite bille contient des cellules qui quand elles sont restreintes en étant emprisonnées dans ladite bille, produisent un facteur qui supprime la prolifération de cellules tumorales, dans laquelle ledit facteur diffuse à travers ladite bille solide d'agarose - collagène enduite.

2. Composition de matière selon la revendication 1, dans laquelle lesdites cellules sont des cellules cancé reuses, de préférence des cellules rénales cancéreuses.

3. Composition de matière selon la revendication 1, dans laquelle ladite bille contient d'environ 10 000 à environ 200 000 cellules et, de préférence, d'environ 30 000 à environ 100 000 cellules.

4. Composition de matière selon l'une quelconque des revendications 1 à 3 pour l'utilisation dans le traitement d'une condition pathologique.

5. Composition de matière selon la revendication 4 dans laquelle ladite condition pathologique est **caractérisée par** une croissance cellulaire anormale.

6. Composition de matière selon la revendication 5, dans laquelle ladite condition pathologique est un cancer.

7. Procédé d'administration d'un facteur à des cellules cultivées in vitro comprenant l'incubation desdites cellules avec la composition de matière selon l'une quelconque des revendications 1 à 3, dans lequel ledit facteur diffuse à travers ladite composition de matière.

8. Procédé de fabrication d'une bille d'agarose-collagène enduite d'agarose qui contient des cellules qui, quand elles sont restreintes en étant emprisonnées dans ladite bille, produisent un facteur qui supprime la prolifération des cellules tumorales qui diffuse à travers ladite bille solide d'agarose - collagène enduite d'agarose, comprenant :
la mise en suspension des cellules qui, quand elles sont restreintes, produisent le facteur dans un matériau contenant du collagène ;
l'addition d'agarose audit matériau contenant du collagène ;
la formation d'une bille semi -solide constituée dudit collagène, d'agarose et des cellules ;
la polymérisation du collagène dans ladite bille semi-solide pour former une bille solide d'agarose -collagène contenant lesdites cellules ; et
le revêtement de ladite bille solide d'agarose -collagène qui contient lesdites cellules avec de l'agarose.

9. Procédé selon la revendication 8, dans lequel lesdites cellules sont des cellules cancéreuses.

10. Procédé selon la revendication 8, comprenant la mise en suspension d'environ 10 000 à environ 200 000 cellules et , de préférence, d'environ 30 000 à environ 100 000 cellules dans ladite solution contenant du collagène.

11. Composition de matière comprenant une bille solide contenant de l'agarose, enduite d'agarose, dans laquelle ladite bille contient des cellules qui, quand elles sont restreintes en étant emprisonnées dans ladite bille, produisent un facteur qui supprime la prolifération des cellules tumorales, dans laquelle ledit facteur diffuse à travers ladite bille solide, contenant de l'agarose, enduite d'agarose.

12. Composition de matière selon la revendication 11, dans laquelle lesdites cellules restreintes sont des cellules cancéreuses et, de préférence, des cellules rénales cancéreuses.

13. Composition de matière selon la revendication 11, dans laquel le ladite bille contient d'environ 10 000 à environ 200 000 cellules et, de préférence, d'environ 30 000 à 100 000 cellules.

14. Composition de matière selon l'une quelconque des revendications 11 à 13 pour l'utilisation dans le traitement d'une condition pathologique.

15. Composition de matière selon la revendication 14 dans laquelle ladite condition pathologique est **caractérisée par** une croissance cellulaire anormale.

16. Composition de matière selon la revendication 15, dans laquelle ladite condi tion pathologique est un cancer.

17. Procédé d'administration d'un facteur à des cellules cultivées in vitro comprenant l'incubation desdites cellules avec la composition de matière selon l'une quelconque des revendications 11 à 13 dans lequel après incubation ledit facteur diffuse à travers ladite composition de matière.

18. Procédé de fabrication d'une bille solide qui comprend de l'agarose et est enduite d'agarose, dans lequel ladite bille solide contient des cellules qui, quand elles sont restreinte s en étant emprisonnées dans ladite bille, produisent un facteur qui supprime la prolifération des cellules tumorales qui diffuse à travers ladite bielle comprenant :
l'addition de l'agarose à une solution qui contient un échantillon de cellules qui sont capables de pr oduire un facteur qui supprime la prolifération des cellules tumorales qui diffuse à travers ladite bille quand lesdites cellules sont restreintes ;
la formation d'une bille semi -solide comprenant ledit agarose et lesdites cellules ;
la polymérisation de l 'agarose dans ladite bille semi-solide pour former une bille solide d'agarose contenant lesdites cellules et de cette façon restreignant lesdites cellules ; et
le revêtement de ladite bille solide contenant de l'agarose contenant les cellules restreintes a vec l'agarose.

19. Procédé selon la revendication 18, dans lequel lesdites cellules sont des cellules cancéreuses.

20. Procédé selon la revendication 18 comprenant la mise en suspension d'environ 10 000 à environ 200 000 cellules et, de préférence, d' environ 30 000 à environ 100 000 cellules dans ladite solution.

21. Composition de matière comprenant une bille solide contenant de l'agarose, enduite d'agarose, dans laquelle ladite bille contient des cellules cancéreuses isolées d'un animal, qui, quand elles sont restreintes en étant emprisonnées dans ladite bille, produisent plus d'un matériau qui supprime la prolifération des cellules tumorales, dans laquelle ledit matériau diffuse à travers ladite bille solide contenant de l'agarose enduite d'agarose.

22. Composition de matière selon la revendication 21, dans laquelle lesdites cellules cancéreuses sont des cellules rénales cancéreuses.

23. Composition de matière selon la revendication 21, dans laquelle ladite bille contient d'environ 10 000 à environ 200 000 cellules.

24. Composition de matière selon la revendication 23, dans laquelle ladite bille contient d'environ 30-000 à environ 100 000 cellules.

25. Composition de matière selon l'une quelconque des revendications 21 -24 pour le traitement d'une condition pathologique laquelle est **caractérisée par** une croissance cellulaire anormale.

26. Composition de matière selon l'une quelconque des revendications 21 -24 pour le traitement d'une condition pathologique laquelle est un cancer.

27. Composition de matière selon l'une quelconque des revendications 21-24, pour l'utilisation dans la suppression de la prolifération des cellules cancéreuses chez un sujet.

28. Utilisation d'une composition de matière selon l'une quelconque des revendications 21-24 pour la fabrication d'un médicament pour l'implantation chez un sujet pour supprimer la prolifération des cellules cancéreuses dans ledit sujet.

29. Procédé de fabrication d'une bille solide qui comprend de l'agarose, et est en duite d'agarose, dans lequel ladite bille solide contient des cellules cancéreuses qui, quand elles sont restreintes en étant emprisonnées dans ladite bille, produisent un matériau qui supprime la prolifération des cellules cancéreuses et diffuse à travers ladite bille, comprenant :
(a) l'addition d'agarose à une solution qui contient un échantillon de cellules cancéreuses isolées d'un animal, qui sont capables de produire un matériau qui supprime la prolifération des cellules cancéreuses qui diffuse à travers ladite bille quand lesdites cellules cancéreuses sont restreintes en étant emprisonnées par la bille ;
(b) la formation d'une bille semi -solide comprenant ledit agarose et lesdites cellules cancéreuses ;
(c) la polymérisation de l'agarose dans ladite bille semi-solide pour former une bille solide d'agarose contenant et, de cette façon, restreignant lesdites cellules cancéreuses, et
(d) le revêtement de ladite bille solide contenant de l'agarose contenant les cellules cancéreuses restreintes avec l'agarose, dans lequel lesdites cellules cancéreuses restreintes produisent plus dudit matériau que quand lesdites cellules cancéreuses ne sont pas emprisonnées dans ladite bille.

30. Procédé selon la revendication 29, dans lequel ladite solution contient d'environ 10 000 à environ 200 000 cellules.

31. Procédé selon la revendication 29 , dans lequel ladite solution contient d'environ 30 000 à environ 100 000 cellules.
